# FASCICULE DE BREVET EUROPEEN

(11) **EP 2 486 389 B1**
(45) Date de publication et mention de la délivrance du brevet: **05.03.2014**
(21) Numéro de dépôt: 10781932.8
(22) Date de dépôt: 08.10.2010
(51) Int. Cl.: G01N 21/31, G01N 21/35, G01N 21/63, G01N 33/00

(54) **CAPTEUR OPTIQUE D'ESPECES CHIMIQUES FONCTIONNANT DANS L'INFRAROUGE.**
IM INFRAROTBEREICH ARBEITENDER OPTISCHER SENSOR FÜR CHEMISCHE SPEZIES
CHEMICAL SPECIES OPTICAL SENSOR OPERATING IN INFRARED

(30) Priorité: 08.10.2009 FR 0904809
(43) Date de publication de la demande: 15.08.2012
(73) Titulaire: Centre National de la Recherche Scientifique CNRS, 75016 Paris (FR)
(72) Inventeur: CAMY, Patrice, F-14000 Caen (FR); DOUALAN, Jean-Louis, F-14980 Rots (FR); NAZABAL, Virginie, 35700 Rennes (FR)
(74) Mandataire: Priori, Enrico
(86) Numéro de dépôt international: PCT/FR2010/000679
(87) Numéro de publication internationale: WO 2011/042628

(56) Documents cités:
- WO-A1-99/13303
- GB-A- 2 102 942
- I.D. AGGARWAL, J.S. SANGHERS: "Development and applications of chalcogenide glass optical fibers at nrl", JOURNAL OF OPTOELECTRONICS AND ADVANCED MATERIAL, vol. 4, no. 3, septembre 2002 (2002-09), pages 665-678, XP002583217, cité dans la demande
- SCHWEIZER T ET AL: "SPECTROSCOPIC DATA OF THE 1.8-, 2.9-, AND 4.3MUM TRANSITIONS IN DYSPROSIUM-DOPED GALLIUM LANTHANUM SULFIDE GLASS", OPTICS LETTERS, OSA, OPTICAL SOCIETY OF AMERICA, WASHINGTON, DC, US, vol. 21, no. 19, 1 octobre 1996 (1996-10-01), pages 1594-1596, XP000630424, ISSN: 0146-9592
- MITSUNORI SAITO ET AL: "CO2 GAS SENSOR USING ACOUSTO-OPTIC BRAGG DIFFRACTION IN AS2SE3 GLASS", REVIEW OF SCIENTIFIC INSTRUMENTS, AIP, MELVILLE, NY, US LNKD- DOI:10.1063/1.1142374, vol. 62, no. 9, 1 septembre 1991 (1991-09-01), pages 2105-2108, XP000262846, ISSN: 0034-6748

## Description

L'invention porte sur un capteur optique d'espèces chimiques, en particulier gazeuses, fonctionnant dans l'infrarouge et plus particulièrement dans l'infrarouge moyen (2 - 5 µm). L'invention porte également sur un procédé de détection optique - et plus précisément spectroscopique - d'espèces chimiques, et notamment du CO₂ gazeux.

De nombreuses espèces chimiques peuvent être détectées en mesurant leur absorption dans l'infrarouge, et plus spécifiquement dans l'infrarouge moyen c'est à dire à des longueurs d'onde comprises entre 2 et 5 µm environ. La détection peut se faire, avantageusement, de manière différentielle, c'est à dire en mesurant à deux longueurs d'onde différentes l'intensité spectrale d'un rayonnement infrarouge ayant traversé une région de détection contenant l'espèce chimique à détecter.

Le rayonnement infrarouge utilisé pour la détection peut être généré par une source thermique de type corps noir : voir par exemple les documents US2007/0279633 et US 6,114,700. Les sources de ce type sont généralement peu satisfaisantes en raison de leur faible brillance, de la difficulté que l'on rencontre à focaliser le rayonnement qu'elles émettent et de la largeur excessive de leur spectre d'émission par rapport à la bande spectrale utile.

Charpentier et al. [1] ont proposé de réaliser un capteur optique de CO₂ gazeux utilisant une source infrarouge, de type thermique, déportée. Dans ce capteur, la région de détection (qui peut être un réservoir géologique, ou tout autre environnement hostile pour les appareils électriques ou électronique) est éloignée de la source, le rayonnement infrarouge y étant transporté par une fibre optique en verre de chalcogénure : en effet, les fibres optiques les plus répandues, en SiO₂ ne sont pas transparentes à des longueurs d'onde supérieures à 2 µm environ. Les fibres en fluorures présentent aussi une longueur d'onde de coupure trop faible. Le détecteur de rayonnement, permettant la mesure de l'intensité spectrale du rayonnement après que ce dernier ait traversé la région de détection, peut également être déporté, et couplé à ladite région de détection par l'intermédiaire d'une deuxième fibre optique en verre de chalcogénure. L'inconvénient de ce système est que les fibres optiques en verre de chalcogénure présentent une atténuation relativement importante, de l'ordre-de 0,1-1 dB/m, ce qui aggrave les inconvénients précités des sources infrarouges de type thermique ([2, 3]).

En pratique, le déport de la source de lumière est ainsi limité à une dizaine de mètres au plus.

Il est également connu (US2005/0269499) d'utiliser des diodes électroluminescentes comme sources de rayonnement infrarouge dans des capteurs optiques d'espèces chimiques. Le spectre du rayonnement émis par ces diodes varie fortement avec la température, ce qui rend la détection différentielle difficile et peu fiable.

L'utilisation de lasers a également été proposée. Des lasers émettant dans l'infrarouge moyen peuvent prévoir, en tant que milieu actif, des ions de terres rares dans une matrice en verre de chalcogénure, éventuellement conformés en fibre optique : voir par exemple [4]. Ces lasers sont complexes à mettre en oeuvre, et couteux.

Le documents WO 99/13303 ainsi que l'article de I. D.Aggarwal et J. S. Sanghers « Develppement and applications of chalogenide glass optical fibers at nrl », Journal of optoelectronics and advanced materials, vol. 4, no. 3, septembre 2002, pages 665 - 678, divulguent l'utilisation d'un élément en verre chalcogénure dopé et pompé optiquement comme source de rayonnement infrarouge incohérent pour la détection spectroscopique de CO₂. Ni la source de pompage ni le détecteur de rayonnement infrarouge ne peuvent être déportés.

L'invention vise à procurer un capteur optique et un procédé de détection optique d'espèces chimiques, et notamment du CO₂ gazeux, ne présentant pas les inconvénients précités de l'art antérieur.

Conformément à un premier aspect de l'invention, ce but peut être atteint par un capteur optique d'espèces chimiques comportant : une source fluorescente de rayonnement infrarouge incohérent, comprenant une matrice en verre de chalcogénure dopée par des ions de terre rare ; au moins un détecteur de rayonnement infrarouge pourvu d'un dispositif de sélection spectrale et agencé pour détecter le rayonnement émis par ladite source fluorescente, une zone de détection d'espèces chimiques étant prévue entre ladite source et ledit détecteur ; et une source de pompage émettant un rayonnement visible ou infrarouge adapté pour exciter la fluorescence desdits ions de terre rare. La source de pompage peut être, typiquement, un laser à semi-conducteur émettant dans le visible ou dans l'infrarouge proche (longueur d'onde inférieure à 1 µm).

Ce dispositif utilise une source de rayonnement infrarouge incohérent, exploitant la fluorescence d'ions de terre rare dispersés dans une matrice en verre de chalcogénure. Une telle source présente une brillance beaucoup plus élevée que les sources thermiques conventionnelles. Son spectre d'émission est suffisamment large pour permettre une détection différentielle des espèces chimiques cibles, sans l'être excessivement comme c'est le cas pour les sources thermiques. Contrairement aux diodes électroluminescentes, une telle source est très peu sensible aux variations de température ambiante, tant que celle-ci reste inférieure à la température de transition du verre, typiquement de l'ordre de 200 - 350 °C, selon la composition de la matrice, et elle est beaucoup plus simple à mettre en oeuvre qu'un laser infrarouge.

Ce capteur comporte également une fibre optique couplant ladite source de pompage à ladite source fluorescente, de manière à permettre leur dissociation spatiale. La source de pompage peut ainsi être déportée et éloignée de l'environnement - potentiellement hostile - dans lequel est réalisée la mesure. Alors que Charpentier et al. [1] avaient proposé de déporter la source de rayonnement infrarouge elle-même, et de transporter ce rayonnement par une fibre en verre de chalcogénure, l'invention propose de ne déporter que la source de pompage et de générer le rayonnement infrarouge in situ par une technique purement optique. L'avantage majeur est que le rayonnement de pompage peut être transporté par une fibre optique classique, en silice, dont les pertes optiques sont très faibles et permettent ainsi un déport de la source pouvant atteindre plusieurs centaines de mètres, voire davantage. La source fluorescente de rayonnement infrarouge n'a pas besoin d'être déportée : il s'agit en effet d'un dispositif purement optique, insensible aux perturbations électromagnétiques et pouvant supporter des températures relativement élevées, comme expliqué plus haut.

Selon différents modes de réalisation de l'invention :
- Ledit détecteur de rayonnement infrarouge peut être pourvu d'un dispositif de sélection spectrale accordable, permettant de mesurer l'intensité spectrale du rayonnement infrarouge ayant traversé ladite zone de détection d'espèces chimiques à au moins deux longueurs d'onde différentes.
- En variante, le capteur peut comporter au moins deux dits détecteurs de rayonnement infrarouge, pourvus chacun d'un dispositif de sélection spectrale accordé à une longueur d'onde différente.
- La matrice en verre de chalcogénure de ladite source fluorescente de rayonnement infrarouge peut être du type Ge-Ga(In)-(Sb)-(Csl)-S(Se,Te), dopée par des ions trivalents d'au moins une terre rare à une concentration supérieure ou égale à 500 parties par million, et de préférence comprise entre 1000 et 3000 parties par million.
- La matrice en verre de chalcogénure de ladite source fluorescente de rayonnement infrarouge peut être dopée par des ions Dy³⁺ ou Pr³⁺ à une concentration supérieure ou égale à 500 parties par million, et de préférence comprise entre 1000 et 3000 parties par million, moyennant quoi ladite source fluorescente de rayonnement infrarouge émet un rayonnement à 4,3 µm.
- Ladite source fluorescente de rayonnement infrarouge peut présenter une émission ayant une largeur spectrale à mi-hauteur comprise entre 200 et 800 nm.
- Ladite source fluorescente de rayonnement infrarouge peut se présenter sous la forme d'un guide d'onde, et de préférence d'une fibre optique. Cela permet d'augmenter la brillance de la source et facilite la focalisation du rayonnement émis sur le détecteur.

Le détecteur de rayonnement infrarouge est à son tour déporté. Là encore, on évite de transporter le rayonnement infrarouge sur des longues distances, ce qui introduirait des pertes élevées que nuiraient à la sensibilité de la détection. Un élément fluorescent (matrice en verre de chalcogénure dopée par des ions de terre rare) pompé par un rayonnement visible ou infrarouge proche, effectue une conversion vers les hautes énergies des photons infrarouges, qui peuvent ensuite être transportés par une fibre optique conventionnelle jusqu'à un détecteur déporté. Ce détecteur, opérant à des longueurs d'onde plus faibles, peut être beaucoup plus efficace que les détecteurs sensibles à l'infrarouge moyen. Plus précisément, ledit détecteur de rayonnement infrarouge comporte :
- un élément fluorescent agencé pour être éclairé par le rayonnement infrarouge émis par ladite source et ayant traversé ladite zone de détection d'espèces chimiques, ledit élément fluorescent comprenant une matrice en verre de chalcogénure dopé par des ions de terre rare dans laquelle lesdits ions, lorsqu'ils se trouvent dans un premier état excité, sont susceptibles d'absorber un photon dudit rayonnement, puis d'émettre un photon à longueur d'onde inférieure ;
- une deuxième source de pompage émettant un rayonnement visible ou infrarouge adapté pour amener lesdits ions de terre rare dans ledit premier état excité ; et
- une deuxième fibre optique couplant ledit élément fluorescent à un détecteur de rayonnement, de manière à permettre leur dissociation spatiale.

Un tel capteur peut comprendre également un deuxième détecteur de rayonnement infrarouge qui comporte : un deuxième élément fluorescent agencé pour être éclairé par le rayonnement infrarouge émis par ladite source et ayant traversé ladite zone de détection d'espèces chimiques, ledit élément fluorescent comprenant une matrice en verre de chalcogénure dopé par des ions de terre rare dans laquelle lesdits ions, lorsqu'ils se trouvent dans un premier état excité, sont susceptibles d'absorber un photon dudit rayonnement, puis d'émettre un photon à longueur d'onde inférieure ; une troisième source de pompage émettant un rayonnement visible ou infrarouge adapté pour amener lesdits ions de terre rare dans ledit premier état excité ; et une troisième fibre optique couplant ledit élément fluorescent à un deuxième détecteur de rayonnement; chacun des deux dits détecteurs de rayonnement infrarouge étant pourvu d'un dispositif de sélection spectrale respectif, accordé à une longueur d'onde différente, pour filtrer le rayonnement infrarouge incident sur l'élément fluorescent respectif.

Ledit ou chaque élément fluorescent peut être couplé à sa source de pompage par une fibre optique respective.

Avantageusement, la matrice en verre de chalcogénure de ladite source fluorescente de rayonnement infrarouge peut être dopée par des ions Dy³⁺ ou Pr³⁺, moyennant quoi ladite source émet un rayonnement à 4,3 µm ; et la matrice en verre de chalcogénure dudit ou de chaque élément fluorescent du capteur peut être dopée par des ions Er ³⁺.

Ledit ou chaque élément fluorescent peut se présenter sous la forme d'un guide d'onde, et de préférence d'une fibre optique.

La ou les fibres optiques reliant ledit ou chaque élément fluorescent à la source de pompage et au détecteur de rayonnement respectifs peuvent être des fibres en silice.

Un autre objet de l'invention est un procédé de détection d'une espèce chimique par mesure de l'absorption, à au moins deux longueurs d'onde distinctes, du rayonnement infrarouge incohérent émis par fluorescence par des ions de terre rare dans une matrice en verre de chalcogénure, caractérisé par l'utilisation d'un capteur tel que décrit ci-dessus.

Avantageusement :
- ledit rayonnement infrarouge incohérent peut présenter une largeur spectrale à mi-hauteur comprise entre 200 et 800 nm et une longueur d'onde de 4,3 µm ; et
- ladite espèce chimique peut être le CO₂ gazeux.

D'autres caractéristiques, détails et avantages de l'invention ressortiront à la lecture de la description faite en référence aux dessins annexés donnés à titre d'exemple et qui représentent, respectivement :
- La figure 1, un premier example d'un capteur optique d'espèces chimiques ;
- La figure 2, un deuxième example d'un capteur optique d'espèces chimiques ;
- La figure 3, un premier mode de réalisation d'un capteur optique d'espèces chimiques ;
- La figure 4, un diagramme de niveaux d'énergie illustrant la conversion vers les hautes énergies des photons infrarouges mise en oeuvre dans le capteur de la figure 3 ;
La figure 5, un capteur optique d'espèces chimiques selon un deuxième mode de réalisation de l'invention ; et

- Les figures 6A et 6B, des graphiques obtenus par des mesures expérimentales démontrant l'utilisation du rayonnement infrarouge incohérent émis par fluorescence par des ions de terre rare dans une matrice en verre de chalcogénure pour la détection optique du CO₂ gazeux.

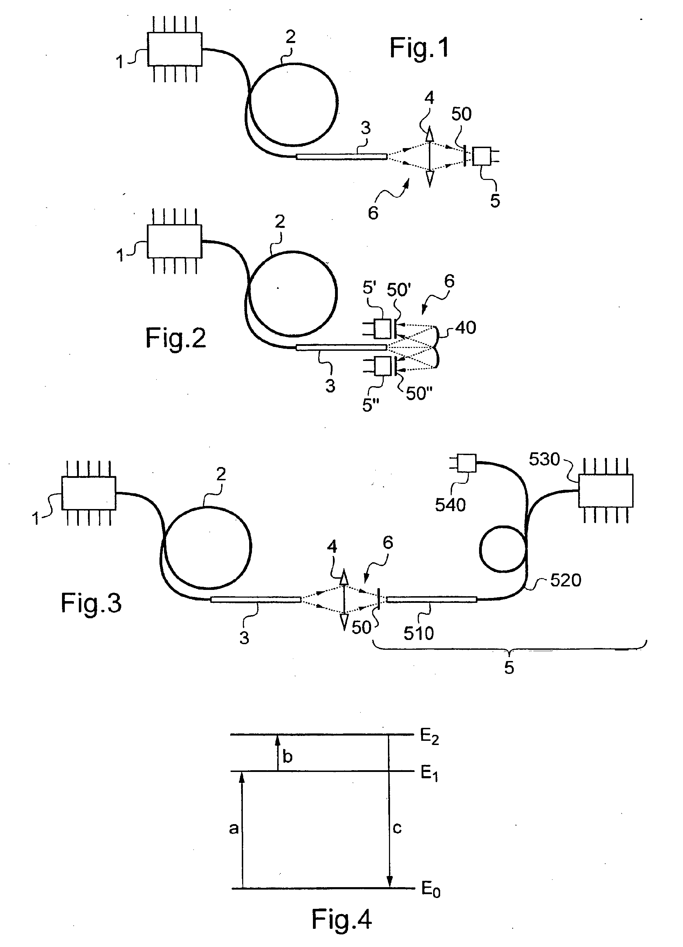

Le capteur de la figure 1, qui ne fait pas partie de l'invention, comporte une source de rayonnement infrarouge se présentant sous la forme d'une fibre optique 3 en verre de chalcogénure, dopée par des ions, en particulier trivalents, de terres rares. La longueur de la fibre peut être de quelques centimètres, par exemple comprise entre 1 et 20 cm.

Au sens large, un chalcogénure est un composé chimique consistant en au moins un atome de chalcogène autre que l'oxygène et au moins un atome d'un élément plus électropositif. Les chalcogènes sont les éléments du groupe 16 du tableau périodique, à savoir : O, S, Se, Te, Po; les oxydes ne sont pas considérés comme des chalcogénures. Les verres de chalcogénures sont des matériaux amorphes constitués de chalcogénures. Ils sont caractérisés par leur transparence dans l'infrarouge, et pour cette raison ils sont utilisés pour réaliser des fibres optiques opérant dans cette région du spectre. Il est connu de doper des verres de chalcogénures avec des terres rares, qui présentent une émission par fluorescence dans la région de transparence de ces matériaux.

La référence [2] décrit d'une manière générale les propriétés et applications des verres de chalcogénures.

Les références [3] et [5] traitent plus spécifiquement des fibres optiques en verres de chalcogénures dopées par des terres rares.

Le document FR 2 771 405 divulgue des compositions de verres de chalcogénures présentant des propriétés optiques particulièrement favorables.

La fibre optique 3 peut être réalisée en un verre de chalcogénure de type Ge-Ga(In)-(Sb)-(Csl)-S(Se, Te), où les éléments entre parenthèses peuvent être présents ou absents. Dans l'exemple de la figure 1, ladite fibre est réalisée en Ge₅Ga₂₀Sb₁₀S₆₅ dopé par des ions de Dy³⁺ à une concentration supérieure à 500 parties par million (ppm), et de préférence comprise entre 1000 et 3000 ppm. Un niveau de concentration aussi élevé permet la réalisation d'une source de rayonnement particulièrement compacte [6]. Des ions de Pr³⁺ pourraient aussi être utilisés en remplacement du Dy³⁺.

Pompée optiquement à 920 nm, la fibre 3 émet une fluorescence présentant un pic à 4,3 µm et une largeur de bande de l'ordre de 250 nm. Cela convient particulièrement bien à la détection du CO₂ gazeux, qui présente justement un pic d'absorption à 4,3 µm.

Le pompage à 920 nm de la fibre 3 est assuré par le laser à semiconducteur 1. Avantageusement, ce laser est déporté, et couplé à la fibre optique en verre de chalcogénure 3 par l'intermédiaire de la première fibre optique 2, de type « conventionnel » et typiquement en silice. Cette fibre 2 présente des pertes extrêmement faibles, et peut donc avoir une longueur de plusieurs dizaines ou centaines de mètres, voire de plusieurs kilomètres.

Le rayonnement infrarouge émis par la fibre 3 traverse une zone de détection contenant les espèces chimiques à détecter (par exemple, du CO₂), puis est focalisé par une lentille 4 sur un détecteur de rayonnement 5. La lentille 4 est réalisée en matériau transparent dans l'infrarouge : chalcogénure, CaF₂, ZnSe, Saphir ; elle pourrait être remplacée par un miroir concave.

Le détecteur 5, de type pyroélectrique ou photoconducteur en PbSe, est équipé d'un filtre spectral accordable 50 (par exemple, le filtre LFP-3950L-337 de Infratec). En agissant sur le filtre spectral 50 il est possible de mesurer l'intensité spectrale du rayonnement incident sur le détecteur 5 à deux longueurs d'ondes différentes (ou plus). Cela permet, selon le principe de la détection spectrale différentielle, de déterminer la concentration d'une espèce chimique absorbante à l'intérieur de la zone 6.

La figure 2 montre un capteur selon un deuxième example, qui ne fait pas partie de l'invention, comportant deux détecteurs de rayonnement infrarouge 5', 5" et deux filtres interférentiels passifs (non accordables) respectifs, 50', 50", transparents à des longueurs d'onde différentes. Le rayonnement issu de la fibre 3 est focalisé sur ces capteurs par un couple de miroirs concaves 40.

La figure 3 illustre un premier mode de réalisation de l'invention du capteur de l'invention, permettant de déporter la quasi-totalité des composants électriques, électroniques ou optoélectroniques, y compris ceux utilisés pour capter le rayonnement infrarouge ayant traversé la région de détection. Dans ce capteur, le détecteur 5 fonctionne sur le principe de la conversion vers les hautes énergies des photons infrarouges. La lentille 4 focalise le rayonnement infrarouge, filtré par le filtre accordable 50, sur une deuxième fibre optique en verre de chalcogénure 510, dopée avec des ions de terre rare. Lorsqu'ils se trouvent dans un premier état excité, d'énergie E₁, ces ions peuvent absorber un photon de ce rayonnement pour atteindre un deuxième état excité, d'énergie E₂. Puis, ils retournent à l'état fondamental E₀ en émettant un photon visible ou dans le proche infrarouge (longueur d'onde supérieure à 1µm, par exemple). Cela est représenté sur la figure 4, où « a » indique le pompage du niveau E₀ au niveau E₁, « b » l'absorption d'un photon infrarouge et « c » l'émission spontanée d'un photon visible ou proche infrarouge.

La fibre optique en verre de chalcogénure 510 peut, par exemple, être dopée par des ions Er³⁺ et être pompée à 980 nm.

Le rayonnement de pompage, émis par le laser à semiconducteur 530, est acheminé vers la fibre en verre de chalcogénure 510 par une fibre optique conventionnelle 520, dite deuxième fibre optique. Cette même fibre optique, ou une fibre séparée, est aussi utilisée pour acheminer le rayonnement émis par fluorescence E₂-E₁ vers un détecteur 540. Le laser 530 et le détecteur 540 peuvent ainsi être déportés. Un autre avantage de ce schéma est que le détecteur 540, sensible dans le proche infrarouge (ou le visible) peut présenter une efficacité supérieure à celle du détecteur 5 pour l'infrarouge moyen. La bande de la transition « b » doit être suffisamment large, et se superposer suffisamment à la bande d'émission de la fibre 3, pour permettre une détection différentielle des espèces chimiques contenues dans la zone 6.

Le filtre 50; du type accordable, peut être situé avant la fibre 510, comme représenté sur la figure. Il n'est pas possible de le disposer juste avant le détecteur 540, car la conversion de fréquence détruit l'information spectrale. Ce filtre accordable demeure donc le seul composant électrique/électronique du détecteur à ne pas être déporté.

Deux possibilités existent pour déporter véritablement tous les éléments électriques du détecteur.

La première consiste à utiliser, à la place du filtre accordable 50, un filtre passif (non accordable). Cela implique de mesurer l'intensité spectrale à une seule longueur d'onde, et renoncer ainsi à une détection différentielle. Néanmoins, cette solution présente l'avantage de la simplicité, et est acceptable lorsqu'il s'agit de détecter une concentration relativement importante d'espèces chimiques, sans qu'une évaluation quantitative de ladite concentration ne soit exigée.

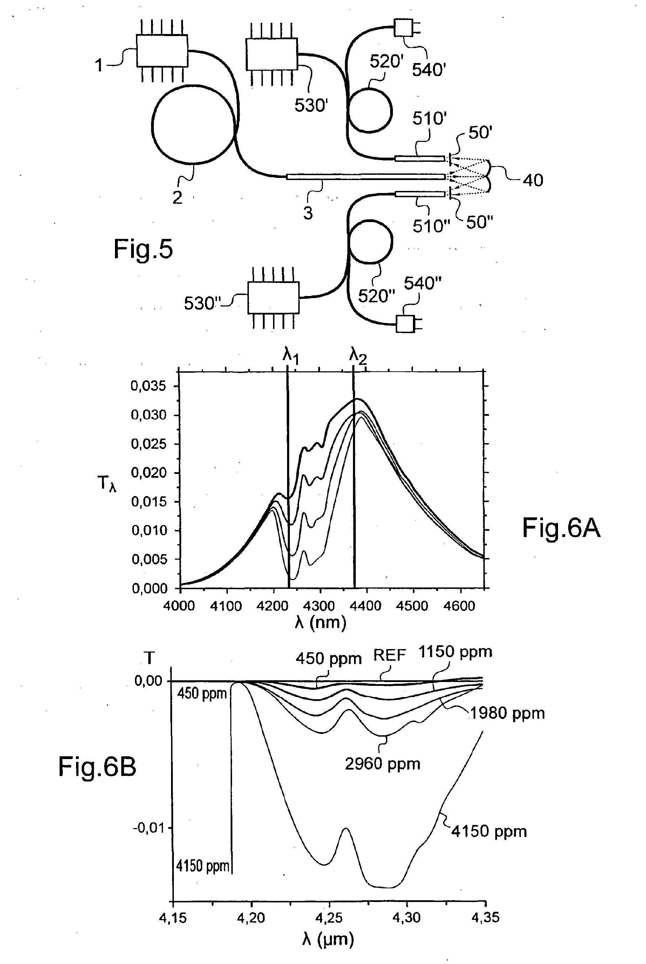

La deuxième possibilité consiste à dédoubler le système de conversion en fréquence, en utilisant deux détecteurs infrarouges 5', 5", c'est à dire deux éléments fluorescents 510', 510" pourvus chacun d'une source de pompage 540', 540" (en fait, une source de pompage commune peut aussi être utilisée), d'un détecteur optique 530', 530" et d'une fibre de couplage 520', 520". Un tel système, dont la complexité demeure acceptable pour la plupart des applications, est illustré sur la figure 5.

La figure 6A montre la variation de l'intensité spectrale I_{λ} du rayonnement infrarouge émis par la fibre 3 de la figure 1 ayant traversé une zone de détection 6 d'une longueur typique de 1 à 10 cm (mais des longueurs plus élevées peuvent également être utilisées) contenant une concentration de CO₂ qui varie entre 450 et 4150 ppm. La figure 6B montre la transmittance T en fonction de la longueur d'onde λ, obtenue en comparant les spectres du rayonnement infrarouge ayant traversée une zone de détection contenant du CO₂ à cinq niveaux de concentration différents avec un spectre obtenu en l'absence de CO₂.

On peut voir que le rapport signal sur bruit est excellent.

Les spectres de la figure 6A ont été obtenus à l'aide d'un monochromateur. Cependant, pour la détection du CO₂ il est suffisant de mesurer I_{λ} à deux longueurs d'onde λ₁ et λ₂ ; comme on peut le voir sur la figure 6A, le rapport I_{λ}(λ₁)/I_{λ} (λ₂), ainsi que la différence I_{λ}(λ₂)-I_{λ} (λ₁), varie avec la concentration de CO₂ et permet donc de la déterminer, moyennant un calibrage préalable.

L'invention peut aussi mettre en oeuvre des techniques d'optique intégrée. Dans ce cas, les fibres optiques, tant en verre de chalcogénures que « conventionnelles », ou au moins certaines d'entre elles, peuvent être remplacées par des guides d'onde planaires. L'utilisation d'éléments fluorescents ne formant pas des guides d'onde peut être envisagée, mais l'utilisation de structures réalisant un confinement du rayonnement est généralement très avantageuse.

### Références :

[1] Charpentier et al. « Infrared monitoring of underground CO2 storage using chalcogenide glass fibers », Optical Materials, Vol. 31, n°3, Janvier 2009, pages 496-500.
[2] D. Lezar « Chalcogenide glasses - survey and progress", Journal of Optoelectronics and Advanced Materials, Vol. 5, n°1, Mars 2003, pages 23 - 34.
[3] J. S. Shangera et al. « Application of chalcogenide glass optical fibers at NRL », Journal of Optoelectronics and Advanced Materials, Vol. 3, n°3, Septembre 2001, pages 627 - 640.
[4] T. Schweitzer et al. «Fabrication and spectroscopy of erbium doped gallium lanthanum sulphide glass fibres for mid-infrared laser applications », Optics Express, Vol. 1, n°4, 18 Août 1997, pages 102 - 107.
[5] B. Cole et al. «Rare-earth doped selenide glasses and fibers for active applications in the near and mid-IR », Journal of Non-Crystalline Solids 256&257 (1999), pages 253 - 259.
[6] V. Moizan, « Etude de l'amplification laser en bande Il dans les fibres de verres chalcogénures », Thèse, Université de Rennes Il, 29 septembre 2008.

## Revendications

1. Capteur optique d'espèces chimiques comportant :
- une source fluorescente de rayonnement infrarouge incohérent (3), comprenant une matrice en verre de chalcogénure dopée par des ions de terre rare ;
- au moins un détecteur de rayonnement infrarouge (5) pourvu d'un dispositif de sélection spectrale (50) et agencé pour détecter le rayonnement émis par ladite source fluorescente, une zone de détection (6) d'espèces chimiques étant prévue entre ladite source et ledit détecteur ; et
- une source de pompage (1) émettant un rayonnement visible ou infrarouge adapté pour exciter la fluorescence desdits ions de terre rare ;
**caractérisé en ce que** :
- il comporte également une première fibre optique (2) couplant ladite source de pompage à ladite source fluorescente ;
et **en ce que** ledit détecteur de rayonnement infrarouge comporte :
- un élément fluorescent (510) agencé pour être éclairé par le rayonnement infrarouge émis par ladite source et ayant traversé ladite zone de détection d'espèces chimiques, ledit élément fluorescent comprenant une matrice en verre de chalcogénure dopé par des ions de terre rare dans laquelle lesdits ions, lorsqu'ils se trouvent dans un premier état excité, sont susceptibles d'absorber un photon dudit rayonnement, puis d'émettre un photon à longueur d'onde inférieure ;
- une deuxième source de pompage (530) émettant un rayonnement visible ou infrarouge adapté pour amener lesdits ions de terre rare dans ledit premier état excité ; et
- une deuxième fibre optique (520) couplant ledit élément fluorescent à un détecteur de rayonnement (540).

2. Capteur optique d'espèces chimiques selon la revendication 1, comprenant également un deuxième détecteur de rayonnement infrarouge qui comporte :
- un deuxième élément fluorescent (510") agencé pour être éclairé par le rayonnement infrarouge émis par ladite source et ayant traversé ladite zone de détection d'espèces chimiques, ledit élément fluorescent comprenant une matrice en verre de chalcogénure dopé par des ions de terre rare dans laquelle lesdits ions, lorsqu'ils se trouvent dans un premier état excité, sont susceptibles d'absorber un photon dudit rayonnement, puis d'émettre un photon à longueur d'onde inférieure ;
- une troisième source de pompage (530") émettant un rayonnement visible ou infrarouge adapté pour amener lesdits ions de terre rare dans ledit premier état excité ; et
- une troisième fibre optique (520") couplant ledit élément fluorescent à un deuxième détecteur de rayonnement (540") ;
chacun des deux dits détecteurs de rayonnement infrarouge (540', 540") étant pourvu d'un dispositif de sélection spectrale respectif (50', 50"), accordé à une longueur d'onde différente, pour filtrer le rayonnement infrarouge incident sur l'élément fluorescent respectif.

3. Capteur optique d'espèces chimiques selon l'une des revendications précédentes, dans lequel ledit ou chaque élément fluorescent (510, 510', 510") est couplé à sa source de pompage (530, 530', 530") par une fibre optique respective (520, 520', 520").

4. Capteur optique d'espèces chimiques selon l'une des revendications précédentes, dans lequel :
- la matrice en verre de chalcogénure de ladite source fluorescente de rayonnement infrarouge (3) est dopée par des ions Dy³⁺ ou Pr³⁺, moyennant quoi ladite source émet un rayonnement à 4,3 µm ; et
- la matrice en verre de chalcogénure dudit ou de chaque élément fluorescent (510, 510', 510") du capteur est dopée par des ions Er³⁺.

5. Capteur optique d'espèces chimiques selon l'une des revendications précédentes, dans lequel ledit ou chaque élément fluorescent (510, 510', 510") se présente sous la forme d'un guide d'onde, et de préférence d'une fibre optique.

6. Capteur optique d'espèces chimiques selon l'une des revendications précédentes, dans lequel la ou les fibres optiques reliant ledit ou chaque élément fluorescent (510, 510', 510") à la source de pompage (530, 530', 530") et au détecteur de rayonnement (540, 540', 540") respectifs sont des fibres en silice.

7. Capteur optique d'espèces chimiques selon l'une des revendications précédentes dans lequel ladite première fibre optique (2) est une fibre en silice.

8. Capteur optique d'espèces chimiques selon l'une des revendications précédentes, dans lequel ledit détecteur de rayonnement infrarouge est pourvu d'un dispositif de sélection spectrale accordable (50), permettant de mesurer l'intensité spectrale du rayonnement infrarouge ayant traversé ladite zone de détection d'espèces chimiques à au moins deux longueurs d'onde différentes.

9. Capteur optique d'espèces chimiques selon l'une des revendications 1 à 7, comportant au moins deux dits détecteurs de rayonnement infrarouge (540',540"), pourvus chacun d'un dispositif de sélection spectrale (50', 50") accordé à une longueur d'onde différente.

10. Capteur optique d'espèces chimiques selon l'une des revendications précédentes, dans lequel la matrice en verre de chalcogénure de ladite source fluorescente de rayonnement infrarouge (3) est du type Ge-Ga(In)-(Sb)-(Csl)-S(Se,Te), dopée par des ions trivalents d'au moins une terre rare à une concentration supérieure ou égale à 500 parties par million, et de préférence comprise entre 1000 et 3000 parties par million.

11. Capteur optique d'espèces chimiques selon l'une des revendications précédentes, dans lequel la matrice en verre de chalcogénure de ladite source fluorescente de rayonnement infrarouge (3) est dopée par des ions Dy³⁺ ou Pr³⁺ à une concentration supérieure ou égale à 500 parties par million, et de préférence comprise entre 1000 et 3000 parties par million, moyennant quoi ladite source fluorescente de rayonnement infrarouge émet un rayonnement à 4,3 µm.

12. Capteur optique d'espèces chimiques selon l'une des revendications précédentes, dans lequel ladite source fluorescente de rayonnement infrarouge (3) présente une émission ayant une largeur spectrale à mi-hauteur comprise entre 200 et 800 nm.

13. Capteur optique d'espèces chimiques selon l'une des revendications précédentes, dans lequel ladite source fluorescente de rayonnement infrarouge (3) se présente sous la forme d'un guide d'onde, et de préférence d'une fibre optique.

14. Procédé de détection d'une espèce chimique par mesure de l'absorption, à au moins deux longueurs d'onde distinctes (λ₁, λ₂), du rayonnement infrarouge incohérent émis par fluorescence par des ions de terre rare dans une matrice en verre de chalcogénure (3), **caractérisé par** l'utilisation d'un capteur d'espèces chimiques selon l'une des revendications précédentes.

15. Procédé selon la revendication 14, dans lequel :
- ledit rayonnement infrarouge incohérent présente une largeur spectrale à mi-hauteur comprise entre 200 et 800 nm et une longueur d'onde de 4,3 µm ; et
- ladite espèce chimique est le CO₂ gazeux.

## Patentansprüche

1. Optischer Sensor für chemische Spezies mit:
- einer fluoreszierenden Quelle inkohärenter Infrarotstrahlung (3) umfassend eine Chalkogenidglasmatrix, die mit Ionen Seltener Erden dotiert ist,
- mindestens einem Infrarotstrahlungsdetektor (5), der mit einer Spektralauswahlvorrichtung (50) versehen und eingerichtet ist, um die von der fluoreszierenden Quelle abgegebene Strahlung zu erfassen, wobei zwischen der Quelle und dem Detektor ein Erfassungsbereich (6) vorgesehen ist; und
- einer Pumpquelle (1), die eine sichtbare oder infrarote Strahlung abgibt, welche ausgelegt ist, um die Fluoreszenz der Ionen Seltener Erden anzuregen;
**dadurch gekennzeichnet, dass**
- er ferner eine erste optische Faser (2) aufweist, die die Pumpquelle mit der fluoreszierenden Quelle koppelt;
und dadurch, dass der Infrarotstrahlungsdetektor aufweist:
- ein fluoreszierendes Element (510), das eingerichtet ist, um von der Infrarotstrahlung, welche von der Quelle abgegeben wird und den Erfassungsbereich für chemische Spezies durchquert hat, beleuchtet zu werden, wobei das fluoreszierende Element eine Chalkogenidglasmatrix umfasst, die mit Ionen Seltener Erden dotiert ist und in der die Ionen, wenn sie sich in einem ersten angeregten Zustand befinden, imstande sind, ein Photon der Strahlung zu absorbieren und anschließend ein Photon mit einer geringeren Wellenlänge abzugeben;
- eine zweite Pumpquelle (530), die eine sichtbare oder infrarote Strahlung abgibt, die ausgelegt ist, um die Ionen Seltener Erden in den ersten angeregten Zustand zu überführen; und
- eine zweite optische Faser (520), die das fluoreszierende Element mit einem Strahlungsdetektor (540) koppelt.

2. Optischer Sensor für chemische Spezies nach Anspruch 1, ferner mit einem zweiten Infrarotstrahlungsdetektor, welcher aufweist:
- ein zweites fluoreszierendes Element (510"), das eingerichtet ist, um von der Infrarotstrahlung, welche von der Quelle abgegeben wird und den Erfassungsbereich für chemische Spezies durchquert hat, beleuchtet zu werden, wobei das fluoreszierende Element eine Chalkogenidglasmatrix umfasst, die mit Ionen Seltener Erden dotiert ist und in der die Ionen, wenn sie sich in einem ersten angeregten Zustand befinden, imstande sind, ein Photon der Strahlung zu absorbieren und anschließend ein Photon mit einer geringeren Wellenlänge abzugeben;
- eine dritte Pumpquelle (530"), die eine sichtbare oder infrarote Strahlung abgibt, die ausgelegt ist, um die Ionen Seltener Erden in den ersten angeregten Zustand zu überführen; und
- eine dritte optische Faser (520"), die das fluoreszierende Element mit einem zweiten Strahlungsdetektor (540") koppelt,
wobei jeder der beiden Infrarotstrahlungsdetektoren (540`, 540") mit einer jeweiligen, auf eine andere Wellenlänge abgestimmten Spektralauswahlvorrichtung (50', 50") versehen ist, um die auf dem jeweiligen fluoreszierenden Element auftreffende Infrarotstrahlung zu filtern.

3. Optischer Sensor für chemische Spezies nach einem der vorhergehenden Ansprüche, bei dem das oder jedes fluoreszierende Element (510, 510', 510") durch eine jeweilige optische Faser (520, 520', 520") mit seiner Pumpquelle (530, 530', 530") gekoppelt ist.

4. Optischer Sensor für chemische Spezies nach einem der vorhergehenden Ansprüche, bei dem:
- die Chalkogenidglasmatrix der fluoreszierenden Quelle infraroter Strahlung (3) mit Dy³⁺- oder Pr³⁺-Ionen dotiert ist, wodurch die Quelle eine Strahlung bei 4,3 µm abgibt; und
- die Chalkogenidglasmatrix des oder jedes fluoreszierenden Elements (510, 510', 510") des Sensors mit Er³⁺-Ionen dotiert ist.

5. Optischer Sensor für chemische Spezies nach einem der vorhergehenden Ansprüche, bei dem das oder jedes fluoreszierende Element (510,510',510") in Form eines Wellenleiters, vorzugsweise einer optischen Faser, vorliegt.

6. Optischer Sensor für chemische Spezies nach einem der vorhergehenden Ansprüche, bei dem die optische(n) Faser(n), die das oder jedes fluoreszierende Element (510, 510`, 514") mit der jeweiligen Pumpquelle (530, 530`, 530") und dem jeweiligen Strahlungsdetektor (540, 540', 540") verbinden, Quarzfasern sind.

7. Optischer Sensor für chemische Spezies nach einem der vorhergehenden Ansprüche, bei dem die erste optische Faser (2) eine Quarzfaser ist.

8. Optischer Sensor für chemische Spezies nach einem der vorhergehenden Ansprüche, bei dem der Infrarotstrahlungsdetektor mit einer abstimmbaren Spektralauswahlvorrichtung (50) versehen ist, mit der sich die spektrale Intensität der Infrarotstrahlung, welche den Erfassungsbereich für chemische Spezies durchquert hat, bei mindestens zwei verschiedenen Wellenlängen bestimmen lässt.

9. Optischer Sensor für chemische Spezies nach einem der Ansprüche 1 bis 7 mit mindestens zwei der Infrarotstrahlungsdetektoren (540', 540"), die jeweils mit einer auf eine andere Wellenlänge abgestimmten Spektralauswahlvorrichtung (50',50") versehen sind.

10. Optischer Sensor für chemische Spezies nach einem der vorhergehenden Ansprüche, bei dem die Chalkogenidglasmatrix der fluoreszierenden Quelle infraroter Strahlung (3) vom Typ Ge-Ga(In)-(Sb)-(Csl)-S(Se,Te) ist und mit dreiwertigen Ionen mindestens einer Seltenen Erde in einer Konzentration von 500 ppm oder mehr, vorzugsweise von zwischen 1000 und 3000 ppm, dotiert ist.

11. Optischer Sensor für chemische Spezies nach einem der vorhergehenden Ansprüche, bei dem die Chalkogenidglasmatrix der fluoreszierenden Quelle infraroter Strahlung (3) mit Dy³⁺- oder Pr³⁺ - Ionen in einer Konzentration von 500 ppm oder mehr, vorzugsweise von zwischen 1000 und 3000 ppm, dotiert ist, wodurch die fluoreszierende Quelle infraroter Strahlung eine Strahlung bei 4,3 µm abgibt.

12. Optischer Sensor für chemische Spezies nach einem der vorhergehenden Ansprüche, bei dem die Emission der fluoreszierenden Quelle infraroter Strahlung (3) eine spektrale Halbwertsbreite zwischen 200 und 800 nm aufweist.

13. Optischer Sensor für chemische Spezies nach einem der vorhergehenden Ansprüche, bei dem die fluoreszierende Quelle infraroter Strahlung (3) in Form eines Wellenleiters, vorzugsweise einer optischen Faser, vorliegt.

14. Verfahren zur Erfassung einer chemischen Spezies durch Messen der Absorption der durch Fluoreszenz von Ionen Seltener Erden in einer Chalkogenidglasmatrix (3) abgegebenen inkohärenten Infrarotstrahlung bei mindestens zwei verschiedenen Wellenlängen (λ₁, λ₂), **gekennzeichnet durch** die Verwendung eines Sensors für chemische Spezies nach einem der vorhergehenden Ansprüche.

15. Verfahren nach Anspruch 14, bei dem:
- die inkohärente Infrarotstrahlung eine spektrale Halbwertsbreite zwischen 200 und 800 nm und eine Wellenlänge von 4,3 µm aufweist; und
- die chemische Spezies gasförmiges CO₂ ist.

## Claims

1. An optical sensor of chemical species comprising:
· a fluorescent source (3) of incoherent infrared radiation comprising a chalcogenide glass matrix doped by rare earth ions;
· at least one infrared radiation detector (5) provided with a spectrum selector device (50) and arranged to detect the radiation emitted by said fluorescent source, a chemical species detection zone (6) being provided between said source and said detector; and
· a pumping source (1) emitting visible or infrared radiation adapted to excite the fluorescence of said rare earth ions;
the sensor being **characterized in that**:
· it also comprises a first optical fiber (2) coupling said pumping source to said fluorescent source;
and **in that** said infrared radiation detector comprises:
· a fluorescent element (510) arranged to be illuminated by the infrared radiation emitted by said source after passing through said chemical species detection zone, said fluorescent element comprising a rare earth ion doped chalcogenide glass matrix in which said ions, when in a first excited state, are capable of absorbing a photon of said radiation, and then of emitting a photon at a shorter wavelength;
· a second pumping source (530) emitting visible or infrared radiation adapted to take said rare earth ions into said first excited state; and
· a second optical fiber (520) coupling said fluorescent element to a radiation detector (540).

2. An optical sensor of chemical species according to claim 1, also including a second infrared radiation detector (5") that comprises:
· a second fluorescent element (510") arranged to be illuminated by the infrared radiation emitted by said source after passing through said chemical species detection zone, said fluorescent element comprising a rare earth ion doped chalcogenide glass matrix in which said ions, when in a first excited state, are capable of absorbing a photon of said radiation, and then of emitting a photon at a shorter wavelength;
· a third pumping source (530") emitting visible or infrared radiation adapted to take said rare earth ions into said first excited state; and
· a third optical fiber (520") coupling said fluorescent element to a second radiation detector (540");
each of said two infrared radiation detectors (540', 540") being provided with a respective spectrum selector device (50', 50") tuned to a different wavelength in order to filter the incident infrared radiation on the respective fluorescent element.

3. An optical sensor of chemical species according to either preceding claim, wherein said or each fluorescent element (510, 510', 510") is coupled to its pumping source (530, 530', 530") by a respective optical fiber (520, 520', 520").

4. An optical sensor of chemical species according to any preceding claim, wherein:
· the chalcogenide glass matrix of said fluorescent source (3) of infrared radiation is doped with Dy³⁺ or Pr³⁺ ions, whereby said source emits radiation at 4.3 µm; and
· the chalcogenide glass matrix of said or each fluorescent element (510, 510', 510") of the sensor is doped with Er³⁺ ions.

5. An optical sensor of chemical species according to any preceding claim, wherein said or each fluorescent element (510, 510', 510") is in the form of a waveguide, and preferably of an optical fiber.

6. An optical sensor of chemical species according to any preceding claim, wherein the or each optical fiber connecting said or each fluorescent element (510, 510', 510") to the pumping source (530, 530', 530") and to the respective radiation detector (540, 540', 540") is a silicon fiber.

7. An optical sensor of chemical species according to any preceding claim, wherein said first optical fiber (2) is a silica fiber.

8. An optical sensor of chemical species according to any preceding claim, wherein said infrared radiation detector is provided with a tunable spectrum selector device (50) enabling the spectral intensity of the infrared radiation that has passed through the chemical species detection zone to be measured at at least two different wavelengths.

9. An optical sensor of chemical species according to any one of claims 1 to 7, including at least two said infrared radiation detectors (540', 540"), each provided with a spectrum selector device (50', 50") tuned to a different wavelength.

10. An optical sensor of chemical species according to any preceding claim, wherein the chalcogenide glass matrix of said fluorescent source (3) of infrared radiation is of the Ge-Ga(In)-(Sb)-(CsI)-S(Se,Te) type, doped by trivalent ions of at least one rare earth at a concentration greater than or equal to 500 ppm, and preferably lying in the range 1000 ppm to 3000 ppm.

11. An optical sensor of chemical species according to any preceding claim, wherein the chalcogenide glass matrix of said fluorescent source (3) of infrared radiation is doped with Dy³⁺ or Pr³⁺ ions at a concentration greater than or equal to 500 ppm, and preferably lying in the range 1000 ppm to 3000 ppm, whereby said fluorescent source of in infrared radiation emits radiation at 4.3 µm.

12. An optical sensor of chemical species according to any preceding claim, wherein said fluorescent source (3) of infrared radiation presents emission with a spectrum width at half maximum lying in the range 200 nm to 800 nm.

13. An optical sensor of chemical species according to any preceding claim, wherein said fluorescent source (3) of infrared radiation is in the form of a waveguide, and preferably of an optical fiber.

14. A method of detecting a chemical species by measuring the absorption at at least two distinct wavelengths (λ₁, λ₂) of incoherent infrared radiation emitted by fluorescence from rare earth ions in a chalcogenide glass matrix (3), the method being **characterized by** the use of a sensor of chemical species according to any preceding claim.

15. A method according to claim 14, wherein:
· said incoherent infrared radiation presents a spectrum width at half maximum lying in the range 200 nm to 800 nm, and a wavelength of 4.3 µm; and
· said chemical species is gaseous CO₂.
